# EUROPEAN PATENT SPECIFICATION

(11) **EP 3 132 266 B1**
(45) Date of publication and mention of the grant of the patent: **12.09.2018**
(21) Application number: 15716510.1
(22) Date of filing: 13.04.2015
(51) Int. Cl.: G01N 33/574

(54) **NEW BIOMARKERS FOR METASTATIC BREAST CANCER**
NEUE BIOMARKER FÜR METASTATISCHEN BRUSTKREBS
NOUVEAUX BIOMARQUEURS DU CANCER DU SEIN MÉTASTATIQUE

(30) Priority: 16.04.2014 EP 14164865
(43) Date of publication of application: 22.02.2017
(73) Proprietor: Deutsches Krebsforschungszentrum, Stiftung des öffentlichen Rechts, 69120 Heidelberg (DE)
(72) Inventor: BURWINKEL, Barbara, 69115 Heidelberg (DE); YANG, Rongxi, 69117 Heidelberg (DE); PENG, Cike, Xuhui District Shanghai 200030 (CN); SCHNEEWEISS, Andreas, 69226 Nussloch (DE)
(74) Representative: Kuttenkeuler, David
(86) International application number: PCT/EP2015/057946
(87) International publication number: WO 2015/158652

(56) References cited:
- BERTRAND DELPECH ET AL: "Serum hyaluronan (hyaluronic acid) in breast cancer patients", INTERNATIONAL JOURNAL OF CANCER, vol. 46, no. 3, 15 September 1990 (1990-09-15), pages 388-390, XP055050210, ISSN: 0020-7136, DOI: 10.1002/ijc.2910460309
- DE LA TORRE M ET AL: "Localization of hyaluronan in normal breast tissue, radial scar, and tubular breast carcinoma", HUMAN PATHOLOGY, SAUNDERS, PHILADELPHIA, PA, US, vol. 24, no. 12, 1 December 1993 (1993-12-01), pages 1294-1297, XP026255607, ISSN: 0046-8177, DOI: 10.1016/0046-8177(93)90262-F [retrieved on 1993-12-01]
- MARÍA D CORTE ET AL: "Expression and Clinical Signification of Cytosolic Hyaluronan Levels in Invasive Breast Cancer", BREAST CANCER RESEARCH AND TREATMENT, KLUWER ACADEMIC PUBLISHERS, BO, vol. 97, no. 3, 10 May 2006 (2006-05-10), pages 329-337, XP019392286, ISSN: 1573-7217, DOI: 10.1007/S10549-005-9130-7

## Description

The present invention pertains to a new method for the diagnosis, prognosis, stratification and/or monitoring of a therapy, of breast cancer in a patient according to the appended set of claims. The method is based on the use of hyaluronic acid and S100P as combined biomarkers in the plasma of breast cancer patients. The new biomarkers of the invention allow diagnosing and even stratifying of breast cancer, in particular metastatic breast cancer. The method provides an interesting alternative to present procedures such as the analysis of the circulating tumor cells (CTC) status. The biomarkers of the invention furthermore allow to monitor therapy success of breast cancer patients and to estimate a patient's prognosis.

### DESCRIPTION

Breast cancer is the most common cancer and the second cause of cancer death in women. Incidence rates of breast cancer in recent years were about 90-100/100,000 in the United States and about 50-70/100,000 in Europe and still incidence rates of the disease is constantly growing worldwide. Breast cancer may generally be divided into several main stages: early, locally advanced, locally recurrent and metastatic. Approximately 450,000 women were diagnosed with the disease in Europe with 140,000 deaths, of which metastasis is the major cause of death. While the 5-year survival prognosis for early stage breast cancer is generally above 60%, this number drops to between 40-60% for advanced breast cancer. The 5-year survival prognosis is generally around 15% for metastatic breast cancer. The most common sites of distant metastasis for breast cancer include lung, liver, bone, lymph nodes, skin and CNS (brain). Once metastatic breast cancer has been diagnosed, a patient may on average expect to live a further 18-24 months. Cure for metastatic breast cancer is unlikely, and the modes of therapy for this systemic disease are largely palliative in nature.

Current therapeutic options for treatment of breast cancer, including metastatic breast cancer, include surgery (e.g. resection, autologous bone marrow transplantation), radiation therapy, chemotherapy (e.g. anthracyclines such as doxorubicin, alkylating agents such as cyclophosphamide and mitomycin C, taxanes such as paclitaxel and docetaxel, antimetabolites such as capecitabine, microtubule inhibitors such as the vinca alkaloid navelbine), endocrine therapy (e.g. antiestrogens such as tamoxifen, progestins such as medroxyprogesterone acetate and megastrol acetate, aromatase inhibitors such as aminoglutethamide and letrozole) and biologics (e.g. cytokines, immunotherapeutics such as monoclonal antibodies). Most commonly metastatic breast cancer is treated by one or a combination of chemotherapy (the most effective drugs including cyclophosphamide, doxorubicin, navelbine, capecitabine and mitomycin C) and endocrine therapy.

Diagnostic options for breast cancer include microscopic analysis of a patient sample-or biopsy-obtained from the affected area of the breast of the patient. The two most commonly used diagnostic approaches are physical examination of the breasts by a medical practitioner and mammography. When these examinations are inconclusive, usually a patient sample of the fluid in the lump is sent for microscopic analysis to help establish the diagnosis. Other options for biopsy include computer tomography (CT), magnet resonance imaging (MRI), an ultrasound examination and a core biopsy or vacuum-assisted breast biopsy, which are procedures in which a section of the breast lump is removed; or an excisional biopsy, in which the entire lump is removed.

During the past decades, many factors have being proved and validated to have predictive value to the prognosis of metastatic breast cancer (MBC) patients. Hormone receptor (estrogen/progesterone receptor, ER/PR) positive and human epidermal growth factor receptor 2 (HER2) negative phenotypes are well documented to be associated with a favorable outcome compared to women with ER and/or PR negative disease. However, these markers are tissue based which can be observed only by biopsy or surgery, and cannot be applied as a real-time monitor for the progress of the disease.

The minimal invasive markers, like blood-based markers, are more appreciated for the monitoring of MBC during clinical application. CA 15-3 is mainly used to monitor patients suffering from MBC. Elevated blood levels of this marker are found in about 70% of patients with advanced disease. However, the variability in CA 15-3 levels in the women with and without breast cancer limits its application as a biomarker for BC. Carcinoembryonic antigen (CEA) is a relevant tumor marker in MBC but with low sensitivity (53.5% to 68.6% in different stage of disease progression).Thus, CEA and CA 15-3 are only recommended to be considered with other prognostic factors.

Recently, enumeration of circulating tumor cells (CTCs) has been considered as a prognostic marker for the patients with MBC. A cardinal cut off of greater than 5 CTCs per 7.5ml of blood has been defined as CTC positive. Compared with patients with negative CTC status, those who are positive displayed shorter progression free survival (11.7 vs. 30.3 weeks) in a multi-center prospective clinical trial. In a prospective study of MBC patients, CTC status was proved to be an independent prognostic factor of progression free survival at a hazard ratio 1.76. Moreover, it is also important to note that about 50% of patients with overt distant metastases are negative for CTCs. This could be partly contributed to the fact of epithelial-mesenchymal transition in CTCs, in which case they can be missed by enumeration techniques that exploit the expression of epithelial markers such as EpCAM or cytokeratin.

Delpech et al. ("Serum hyaluronan (hyaluronic acid) in breast cancer patients", 1990) observed a significant difference of serum hyaluronan (hyaluronic acid, HA) in metastatic cancer patients compared to non-metastatic cancer patients.

De la Torre et al.: (in: "Localization of hyaluronan in normal breast tissue, radial scar, and tubular breast carcinoma", 1993) observed an increased amount of HA in tubular breast carcinoma and radial scar as compared to normal breast tissue specimens.

Corte el al. (in: "Expression and clinical significance of cytosolic hyaluronan levels in invasive breast cancer", 2006) report a significant positive correlation between intratumoral HA levels and survival of patients.

In view of the above described background art, the objective of the present invention is to provide a simple and minimal invasive but specific and sensitive test system for the diagnosis or monitoring of breast cancer, and in particular of metastatic breast cancer. Furthermore the present invention seeks to provide diagnostic strategies that allow to directly monitor the ongoing treatment success of breast cancer therapy in the clinic, and to evaluate the prognosis for a breast cancer patient receiving therapy.

The above problem is solved in a first aspect by a method for the diagnosis, prognosis, stratification, and/or monitoring of a therapy, of breast cancer in a patient, comprising the steps of
a. Providing a blood sample of said patient,
b. Determining the level of hyaluronic acid (HA) and S100P in said blood sample, and
c. Comparing the level HA and S100P as determined in b. with a control,
wherein an increase in the level of HA and S100P in said blood sample compared to said control is indicative for the presence of breast cancer in said patient.

Most preferred in context of the invention is metastatic breast cancer.

A "diagnosis" or the term "diagnostic" in context of the present invention means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

The term "prognosis" refers to a forecast as to the probable outcome of the disease as well as the prospect of recovery from the disease as indicated by the nature and symptoms of the case. Accordingly, a negative or poor prognosis is defined by a lower post-treatment survival term or survival rate. Conversely, a positive or good prognosis is defined by an elevated post-treatment survival term or survival rate. Usually prognosis is provided as the time of progression free survival or overall survival.

The term "stratification" for the purposes of this invention refers to the advantage that the method according to the invention renders possible decisions for the treatment and therapy of the patient, whether it is the hospitalization of the patient, the use, effect and/or dosage of one or more drugs, a therapeutic measure or the monitoring of a course of the disease and the course of therapy or etiology or classification of a disease, e.g., into a new or existing subtype or the differentiation of diseases and the patients thereof. Particularly with regard to breast cancer, "stratification" means in this context a classification of a breast cancer disease of an individual patient with regard of the metastatic status, or the presence or absence of circulating tumor cells. Also, the term "stratification" covers in particular the risk stratification with the prognosis of an outcome of a negative health event.

The term "monitoring a therapy" means for the purpose of the present invention to observe disease progression in a patient who receives a breast cancer therapy. In other words, the patient during the therapy is regularly monitored for the effect of the applied therapy, which allows the medical practitioner to estimate at an early stage during the therapy whether the prescribed treatment is effective or not, and therefore to adjust the treatment regime accordingly.

As used herein, the term "subject" or "patient" refers to any animal (e.g., a mammal), including, but not limited to, humans, non-human primates, rodents, and the like, which is to be the recipient of a particular treatment. Typically, the terms "subject" and "patient" are used interchangeably herein in reference to a human subject. As used herein, the term "subject suspected of having cancer" refers to a subject that presents one or more symptoms indicative of a cancer (e.g., a noticeable lump or mass). A subject suspected of having cancer may also have one or more risk factors. A subject suspected of having cancer has generally not been tested for cancer. However, a "subject suspected of having cancer" encompasses an individual who has received an initial diagnosis (e.g., a CT scan showing a mass) but for whom the subtype or stage of cancer is not known. The term further includes people who once had cancer (e.g., an individual in remission), and people who have cancer and are suspected to have a metastatic spread of the primary tumor.

The term "cancer" and "cancer cells" refers to any cells that exhibit uncontrolled growth in a tissue or organ of a multicellular organism. The term "breast cancer" is understood to mean any cancer or cancerous lesion associated with breast tissue or breast tissue cells and can include precursors to breast cancer, for example, atypical ductal hyperplasia or non-atypical hyperplasia. Breast cancer a disease in which a primary tumor or multiple individual primary tumors exist in the breast or breasts. Generally, this means that no cancerous cell has (yet) become separated from the primary tumor in the breast, and has not spread to a "distinct location". The term "tumor" refers to an abnormal benign or malignant mass of tissue that is not inflammatory and possesses no physiological function.

As used herein, the term "metastatic breast cancer" is to be understood as a disease in which at least one transformed, i.e. cancerous cell from a primary tumor of the breast has become separated from the primary tumor and has continued to grow into a tumor at a location distinct from that of the primary tumor (hereinafter "distinct location"). The distinct location may for example be within the same breast as that in which the primary tumor is located (ipsilateral breast) or within the other breast (contralateral breast). Further examples include metastasis in one or more lymph nodes, whether these are movable or fixed, ipsilateral or contralateral to the primary tumor, supraclavicular, axillary or otherwise located. Within the context of the TNM tumor classification, a "metastatic breast cancer" as used herein would include i.e. all tumors whose staging includes M=1 (i.e. Stage IV breast cancer), i.e. all tumors for which any degree of metastasis exists to distant locations such as for example lung, liver, bone, lymph nodes, skin, brain and/or a distinct location within an ipsilateral and/or contralateral breast.

It should be noted that the term "metastatic breast cancer" as used herein does not imply that said metastasis existing at a "distinct location" must have arisen from any one particular primary tumor of the breast. That is to say, the origin of the metastasis at the "distinct location" is immaterial to the designation of the disease as "metastatic breast cancer" as long as the primary tumor giving rise to the metastasis originated in the breast tissue. For this purpose, the term "breast tissue" is to be understood as including the lobules and the ducts of the breast, i.e. the tissue which most commonly gives rise to tumors of the breast.

The term "biological sample" as used herein refers to a sample that was obtained and may be assayed for HA and S100P as disclosed with the present invention, or their gene expression. The sample can include a biological fluid (e.g., blood, cerebrospinal fluid, urine, plasma, serum), tissue biopsy, and the like. The sample can also include a tissue sample, for example, tumor tissue, and may be fresh, frozen, or archival paraffin embedded tissue. Samples for the purposes of the present invention are blood samples.

A "biomarker" or "marker" in the context of the present invention refers to an organic biomolecule, particularly a polypeptide or polysaccharide, which is differentially present in a sample taken from patients having a certain condition as compared to a comparable sample taken from subjects who do not have said condition (e.g., negative diagnosis, normal or healthy subject, or non-metastatic breast cancer patients, depending on whether the patient is tested for breast cancer, or metastatic breast cancer). For examples, a marker can be a polypeptide or polysaccharide (having a particular apparent molecular weight) which is present at an elevated or decreased level in samples of metastatic breast cancer patients compared to samples of patients with a negative diagnosis, or non-metastatic breast cancer patients.

The term "determining the level of' a biomarker in a sample, control or reference, as described herein shall refer to the quantification of the presence of said biomarkers in the testes sample. For example the concentration of the biomarkers in said samples may be directly quantified via measuring the amount of protein/polypeptide/polysaccharide as present in the tested sample. However, also possible is to quantify the amount of biomarker indirectly via assessing the gene expression of the encoding gene of the biomarker, for example by quantification of the expressed mRNA encoding for the respective biomarker. The present invention shall not be restricted to any particular method for determining the level of a given biomarker, but shall encompass all means that allow for a quantification, or estimation, of the level of said biomarker, either directly or indirectly. "Level" in the context of the present invention is therefore a parameter describing the absolute amount of a biomarker in a given sample, for example as absolute weight, volume, or molar amounts; or alternatively "level" pertains to the relative amounts, for example and preferably the concentration of said biomarker in the tested sample, for example mol/l, g/l, g/mol etc. In preferred embodiments the "level" refers to the concentration of the tested biomarkers in g/l.

"Increase" of the level of a biomarker in a sample compared to a control shall in preferred embodiments refer to statistically significant increase in preferred aspects of the invention.

In course of the present invention a set of breast cancer patient related data including a full set of clinical characteristics and follow-up data was investigated, aiming to find potential markers for detection and prognostication of breast cancer. Surprisingly hyaluronic acid (HA) and S100P protein were identified as interesting diagnostic markers that a significantly differentially expressed in the plasma of the observed patients. Plasma HA/S100P level therefore serves as a prognostic marker independent of the CTC status according to the present invention, and is even better than the actual FDA approved CTC based test. Since the biomarkers of the invention were detected via immune-methodologies on the molecule level, the present invention is in particular advantageous because of the simplicity of an immuno based assay, which is minimal invasive, and still very sensitive even if only minimal amounts of sample material can be provided. The methods as described herein are also cost efficient.

S100P is a 95-amino-acid protein which is a member of S100 family. This Ca2+ binding proteins are involved in many intra- and extracellular activities, including protein phosphorylation and enzyme activation, gene transcription, cell proliferation and differentiation, and affect cytoskeleton dynamics. S100P can initiate tumor cell proliferation and survival against chemotherapeutic agent like 5-flurouracil by binding Ca2+ and activation of mitogen-activated protein kinase (MAPK) pathway.

Hyaluronic acid (HA) is an anionic, nonsulfated glycosaminoglycan distributed widely throughout connective, epithelial, and neural tissues. It is unique among glycosaminoglycans in that it is nonsulfated, forms in the plasma membrane instead of the Golgi, and can be very large, with its molecular weight often reaching the millions. It is a major macropolysaccharide in the extracellular matrix of connective tissues, which has been experimentally proved associated with cell proliferation and migration.

The breast cancer that is in accordance with the invention subject of the method for the diagnosis, prognosis, stratification and/or monitoring is metastatic breast cancer or recurrent breast cancer, or circulating tumor cell (CTC) positive breast cancer.

The control as used in the method of the herein described invention corresponds to the level of HA and S100P in a control sample from a healthy individual, or a control sample from non-metastatic breast cancer patient, or a control sample from a circulating tumor cell (CTC) negative breast cancer patient. Thus, the control shall always correspond to the level of the biomarker to be determined, in a control sample that is comparable to the blood sample as used in the method of the invention.

One preferred embodiment of the invention pertains to the above described method which is applied for the diagnosis, prognosis, stratification and/or monitoring of a therapy, of CTC positive breast cancer in a patient.

Epithelial cells that are exfoliated from solid tumors have been found in very low concentrations in the circulation of patients with advanced cancers of the breast and the presence or relative number of these cells in blood has been correlated with overall prognosis and response to therapy. These exfoliated epithelial cells, also called circulating tumor cells, may be an early indicator of tumor expansion or metastasis before the appearance of clinical symptoms.

Circulating tumor cells typically have a short half-life of approximately one day, and their presence generally indicates a recent influx from a proliferating tumor. Therefore, circulating tumor cells represent a dynamic process that may reflect the current clinical status of patient disease and therapeutic response. In some cases, circulating tumor cells can lyse and/or undergo apoptosis, leaving fragments of circulating tumor cells.

The method of the invention comprises that said control corresponds to the level of HA and S100P in a sample of a CTC negative breast cancer patient, and wherein an increase of the level of HA and S100P in said blood sample compared to said control is indicative for CTC positive breast cancer in said patient. Therefore, in this context the tested patient is already diagnosed to have primary breast cancer or metastatic breast cancer, however, who is suspected to have CTC positive breast cancer. Since the CTC positive breast cancer phenotype is an important prognostic factor for the time of progression free survival and overall survival of a breast cancer patient, the diagnosis of the CTC phenotype by the method of the invention is advantageous for medical practitioners in the clinic as it avoids laborious CTC enumeration.

The object of the present invention is furthermore solved by a method for evaluating the treatment success of a breast cancer patient who received a breast cancer treatment, the method comprising the steps of
a. Providing a blood sample of said breast cancer patient who received a breast cancer treatment,
b. Determining the level of HA and S100P in said blood sample, and
c. Comparing the level of HA and S100P as determined in b. with a reference,
wherein a decrease of at least 20%, preferably 25%, preferably 30%, more preferably 35% to 45% of the level of HA and S100P in said blood sample compared to said reference is an indication for the patient's response to said treatment.

The "reference" in the context of the here described aspect of the invention corresponds to the level of HA and S100P in a provided blood sample obtained from said patient before receiving said treatment. Thereby the reference corresponds to an untreated control.

Alternatively the method may include that said reference is provided by the additional method steps, comprising (i.) providing a second bood sample of said patient obtained before said patient received said treatment, (ii.) determining the level of HA and S100P in said second blood sample, wherein said level of HA and S100P in said second blood sample constitutes said reference. Therefore, the second blood sample was obtained at a time the patient had not yet received said treatment to be evaluated by the method, whereas the blood sample was obtained at a time after said treatment to be evaluated was started, in particular at a time during or at or after completion of said treatment to be evaluated.

Yet another aspect of the invention pertains to a method for the prognosis of a breast cancer patient, comprising
a. Providing a blood sample of said breast cancer patient,
b. Determining the level of HA and S100Pin said blood sample,
wherein an elevated level of HA and S100P in said blood sample is an indication for a reduced time of progression free survival (PFS) and/or overall survival (OS).

Of course in case other biomarkers are used in context of the above method also a decrease of said level in said sample may be indicative for a reduced time of progression free survival (PFS) and/or overall survival (OS). If decreased levels of the biomarker are associated with poor prognosis, then also a decrease of said biomarker in said sample may be indicative for a reduced time of progression free survival (PFS) and/or overall survival (OS).

In context of the invention, an elevated level of HA is a concentration of HA in said blood sample of at least about 250 ng/ml, and/or wherein an elevated level of S100P in said blood sample is a concentration of at least about 7 ng/ml. However, the absolute concentration levels strongly dependent on the sample handling and quantification method used. The above levels apply for a sample handling corresponding to the methods used in context of the examples. Therefore, the skilled in the art appreciates that without being confronted by undue experimentation, new reference values must be determined in accordance with a change of a sample treatment protocol.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure. In particularly preferred embodiments of the invention the term "about" may refer to a deviation of the respective numeric value of a maximum of 20% of the numerical value, however more preferred is 15%, 10%, 5% and most preferred is 4%, 3%, 2%, and most preferred is 1%.

In a preferred embodiment said blood sample is selected from the group consisting of a plasma or serum sample.

In another preferred embodiment of the herein described invention said patient is a mammal, preferably a human, more preferably a human patient diagnosed with breast cancer, or a human patient diagnosed with metastatic breast cancer. Since the invention allows diagnosing not only breast cancer, but also metastatic breast cancer end even CTC positive breast cancer, the patient/subject of the invention may vary depending on which of the above stages of the disease is diagnosed, prognosed, stratified or tested. The person of skill is aware that a patient already diagnosed with metastatic breast cancer would not be tested for the general presence of breast cancer as such. However, of course this shall not be interpreted as restriction of the scope of the invention as also already established diagnoses may be verified with any one of the herein described methods of the invention.

In all aspects and embodiments of the present invention it may be preferred that the level of HA and S100P in said sample is determined by means of a nucleic acid detection method or an immunological detection method. However, nucleic acid detection methods are only applicable where an expressed protein is the biomarker. Generally all means shall be comprised by the present invention which allow for a quantification of the expression of such proteins. Therefore also promoter analysis, and procedures assessing the epigenetic status of a gene locus encoding a protein biomarker of the invention are comprised by the herein described invention. Further, HA for example as a polysaccharide molecule is not genetically expressed and therefore is preferably detected using method that allows quantifying a molecule based on its structure (Antibodies) or via its mass. Alternatively, the presence or expression of enzymes regulating HA metabolism may be assessed which is indicative of the presence or level of HA.

Detection methods that are preferred in context of the herein described invention the level of HA and S100P in said sample is determined by means of a detection method selected from the group consisting of mass spectrometry, mass spectrometry immunoassay (MSIA), antibody-based protein chips, 2-dimensional gel electrophoresis, stable isotope standard capture with anti-peptide antibodies (SISCAPA), high-perfomrance liquid chromatography (HPLC), western blot, cytometry bead array (CBA), protein immuno-precipitation, radio immunoassay, ligand binding assay, and enzyme-linked immunosorbent assay (ELISA), preferably wherein said protein detection method is ELISA. Suitable alternative detection methods for quantification of a biomarker of the invention are known to the skilled artisan.

The methods of the herein described invention are preferably in-vitro methods, preferably in-vitro methods that do not comprise any method steps performed at the human or animal body.

In yet another aspect, the invention provides a diagnostic kit according to the appended set of claims for aiding a diagnosis of breast cancer, or metastatic breast cancer, or CTC positive breast cancer, wherein the diagnostic kit can be used to detect HA and S100P.

For example, the diagnostic kit can be used to detect whether HA and S100P are differentially present in samples of a patient having breast cancer, or metastatic breast cancer, or CTC positive breast cancer, and normal patients. The diagnostic kit of the invention has many applications. For example, the diagnostic kit can be used to differentiate if a subject has breast cancer, or metastatic breast cancer, or CTC positive breast cancer, or has a negative diagnosis, thus aiding a breast cancer diagnosis. In another example, the diagnostic kit can be used to identify compounds that modulate expression of HA and S100P in *in vitro* breast cancer cells or *in vivo* animal models for breast cancer.

Optionally, the diagnostic kit can further comprise instructions for suitable operational parameters in the form of a label or a separate insert. For example, the diagnostic kit may have standard instructions informing a consumer how to wash the probe after a sample of plasma is contacted on the probe.

The diagnostic kit can further comprise (a) an antibody that specifically binds to HA and S100P; and (b) a detection reagent. Such diagnostic kits can be prepared from the materials described above, and the previous discussion regarding the materials (e.g., antibodies, detection reagents, immobilized supports, etc.) is fully applicable to this section and need not be repeated.

The diagnostic kit may optionally further comprise a standard or control information so that the test sample can be compared with the control information standard to determine if the test amount of a marker detected in a sample is a diagnostic amount consistent with a diagnosis of cancer, preferably breast cancer.

The diagnostic kit of the invention is a diagnostic kit comprising means for quantifying the level of HA and S100P. Such means for quantifying is for example at least one antibody, preferably wherein the antibody is a monoclonal antibody, such as a monoclonal antibody that specifically binds to HA or S100P. Such antibodies are known in the art and commercially available.

The diagnostic kit of the invention can also comprise a first antibody specifically binding to HA and a second antibody specifically binding S100P, preferably wherein said first and second antibodies are monoclonal antibodies.

It is particularly preferred that the diagnostic kit of the invention comprises means to perform an ELISA.

The object of the present invention is solved in a further aspect by a use of HA and S100P as biomarker in a screening method for breast cancer therapeutics.

The screening may be performed in a cell-culture or an animal model, for example a mouse or rat breast cancer model. In said screening method preferably the use of an animal suffering from breast cancer is included. The progression of the breast cancer in said model based on the biomarkers of the present invention may be monitored in response to contacting said model with a candidate breast cancer therapeutic or therapeutic regime. Therefore, a use is preferred wherein in said screening method a test-compound causes a decrease of the amount of HA and S100P, said test-compound is a candidate to be used as a breast cancer therapeutic.

The present invention will now be further described in the following examples with reference to the accompanying figures and sequences, nevertheless, without being limited thereto. In the Figures and Sequences:
- Figure 1:: (A) Plasma hyaluronic acid and S100P level in health controls, primary breast cancer patients and metastatic breast cancer patients; (B) Metastatic breast cancer patients were further stratified by their CTC status.
- Figure 2:: ROC analysis of plasma HA/S100P and subgroups of breast cancer patients. (A) CTC+ metastatic breast cancer patients against health controls (A) and primary breast cancer patients (B); Metastatic breast cancer patients against health controls(C) and primary breast cancer patients (D).
- Figure 3:: (A) Kaplan-Meier curves of progression free survival, stratified by plasma HA/S100P level; (B) Kaplan-Meier curves of progression free survival, stratified by CTC status.
- Figure 4:: (A) Kaplan-Meier curves of overall survival, stratified by plasma HA/S100P level; (B) Kaplan-Meier curves of overall survival, stratified by CTC status.
- Figure 5:: Plasma HA and S100P levels before and after 1 complete cycle of chemotherapy in metastatic breast cancer patients.
- Figure 6:: ROC analysis of plasma HA and S100P level changes, as well as their combination, in indicating treatment outcome of metastatic breast cancer patients. The changes of CTC numbers was also plotted to compare the discrimination power.
- Figure 7:: Prognostic value of after treatment plasma HA/S100P level in metastatic breast cancer patients. (A) and (B): Kaplan-Meier curves of progression free survival stratified by plasma HA/S100P level and CTC status; (C) and (D): Kaplan-Meier curves of overall survival stratified by plasma HA/S100P level and CTC status.
- Figure 8:: Plasma HA level of patients from the validation cohort and its prognostic value for MBC patients. (A) Plasma HA levels in MBC and PBC patients in the validation cohort; (B) The discrimination power of plasma HA levels in distinguishing MBC from PBC patients; (C, D) The prognostic value of plasma HA level for the PFS and OS of MBC patients, respectively, in the validation cohort as estimated by Kaplan-Meier curves. Low HA: ≤ 250 ng/mL HA in plasma; High HA: > 250 ng/mL HA in plasma. CTC-neg: < 5 CTCs in 7.5 mL periphery blood; CTC-pos: ≥ 5 CTCs in 7.5 mL peripheral blood.

### EXAMPLES

### Materials and Methods

60 healthy controls, 48 primary breast cancer (PBC) patients and 212 MBC patients were included and plasma were collected by centrifugation at 1300g for 20 min at 10°C, followed by additional centrifugation of supernatant at 15,500 g for 10 min at 10°C. Samples were snap-frozen and stored at -80°C for HA/S100P measurement. CTCs were enumerated in MBC patient by CellSearch® system (Veridex, LLC, Raritan NJ), an FDA approved prognostic test. Based on the CTC numbers, patients were classified as CTC positive (≥5 intact CTCs/7.5ml blood) or CTC-negative (< 5 intact CTCs/7.5ml blood). Within 2 hours after the blood collection, plasma was extracted All the cases were followed up and the disease progression was recorded. 66 CTC+ patients were further followed up during their cancer treatment. After 1 complete cycle of chemotherapy, the response to the treatment was investigated by radiological examination and the blood sample was taken again for CTC enumeration and plasma was extracted for HA/S100P quantification. HA/S100P in plasma was measured by ELISA, which is much easier than the capturing and counting of CTCs. Statistical analysis was conducted using SPSS 17.0 (Wilcoxon Rank test, logistic regression, Cox regression and ROC analysis).

### Example 1: The plasma HA/S100P level can differentiate MBC patients from healthy controls and non-MBC patients

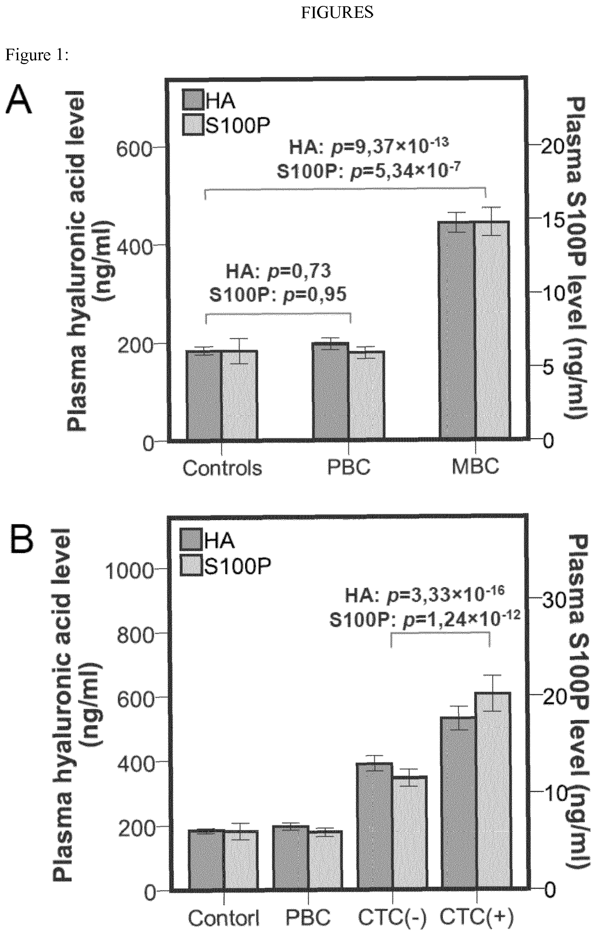

To investigate plasma HA/S100P level in different phases of disease, plasma samples were assayed in 60 healthy volunteers, 48 PBC patients and 212 MBC patients. Compared with controls (HA median[IQR]: 176,9 ng/ml [144,1-216,9]; S100P median[IQR]: 4,5 ng/ml [3,4-7,3]), the PBC patients had similar plasma HA/S100P level (HA median[IQR]: 199,9 ng/ml [138,4-253,1], p=0,73; S100P median[IQR]: 5,9 ng/ml [4,0-7,7], p=0,95), while the MBC patients had more than two-fold higher plasma HA/S100P level (HA median[IQR]: 371,9 ng/ml [217,0-616,8], p=9,37×10-13; S100P median[IQR]: 5,9 ng/ml [4,0-7,7], p= 5,34×10-7, Figure 1A and Table 1). We further stratified MBC patients into CTC+ and CTC-, based on their CTC numbers. Plasma HA/S100P levels were found to elevated in CTC+ patients (HA median[IQR]: 442,7 ng/ml [343,1-596,1]; S100P median[IQR]: 14,1 ng/ml [7,7-29,4]), compared with CTC- MBC patients (HA median[IQR]: 253,5 ng/ml [184,4-629,3], p=3,33×10-16; S100P median[IQR]: 8,1 ng/ml [5,0-13,85], p=1,24×10-12, Figure 1B and Table 1).

**Table 1: Plasma HA/S100P level in healthy controls, PBC and MBC patients**

| Group | N | HA (Median, IQR, ng/ml) | *p* (HA) | S100P (Median, IQR, ng/ml) | *p* (S100P) |
|---|---|---|---|---|---|
| Control | 60 | 176,9 (144,1-216,9) | - | 4,5 (3,4-7,3) | - |
| PBC | 48 | 199,9 (138,4-253,1) | 0,73 | 5,9 (4,0-7,7) | 0,95 |
| MBC | 212 | 371,9 (217,0-616,8) | 9,37×10⁻¹³ | 9,6 (5,7-18,3) | 5,34× 10⁻⁷ |
| CTC - | 133 | 253,5 (184,4-629,3) | 1,73×10⁻⁸ | 8,1 (5,0-13,85) | 1,79×10⁻³ |
| CTC+ | 79 | 442,7 (343,1-596,1) | 3,33×10⁻¹⁶ | 14,1 (7,7-29,4) | 1,24×10⁻¹² |

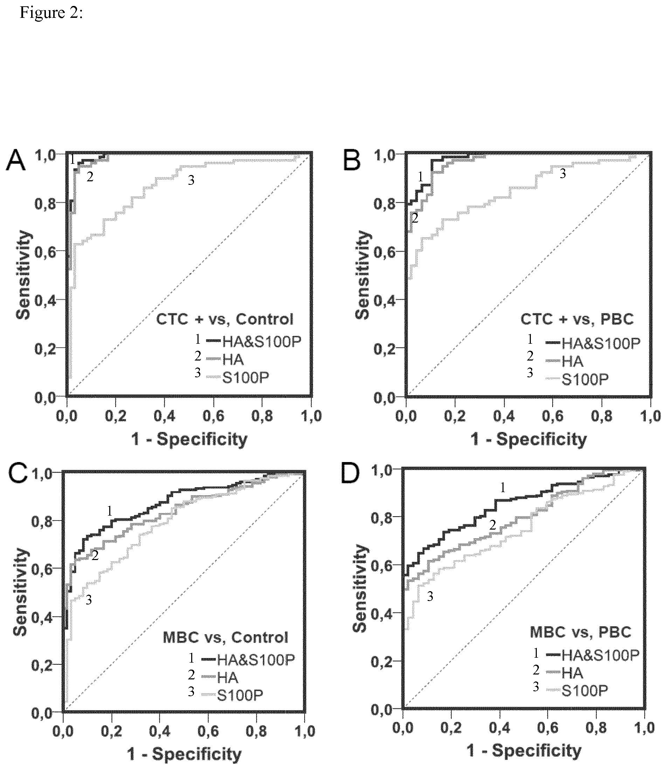

ROC analysis was conducted to study the discrimination power of plasma HA/S100P in detecting sub groups in BC patients. Plasma HA/S100P level presented AUC of 0,99 (95% CI: 0,96-1,00, Figure 2A) in detecting CTC+ MBC cases against health controls, while it showed AUC of 0,98 (95% CI: 0,96-0,99, Figure 2B) in detection of CTC+ patients against PBC patients. Plasma HA/S100P also had a significant discrimination power in detecting MBC patients against health controls (AUC: 0,87, 95% CI: 0,83-0,91, Figure 2C) and primary breast cancer patients (AUC:0,85, 95% CI: 0,80-0,90, Figure 2D). As shown in Figure 2, this combined HA/S100P indicator presented a better discrimination power than use HA or S100P alone, as the AUC of combined indicator were always large than the single marker.

Thus, the plasma HA/S100P level can efficiently distinguish MBC patients from non-metastasis individuals and is a promising marker for detection of BC metastasis and recurrence.

### Example 2: The combination of plasma S100P and HA level as a marker for the progression free survival of MBC patients

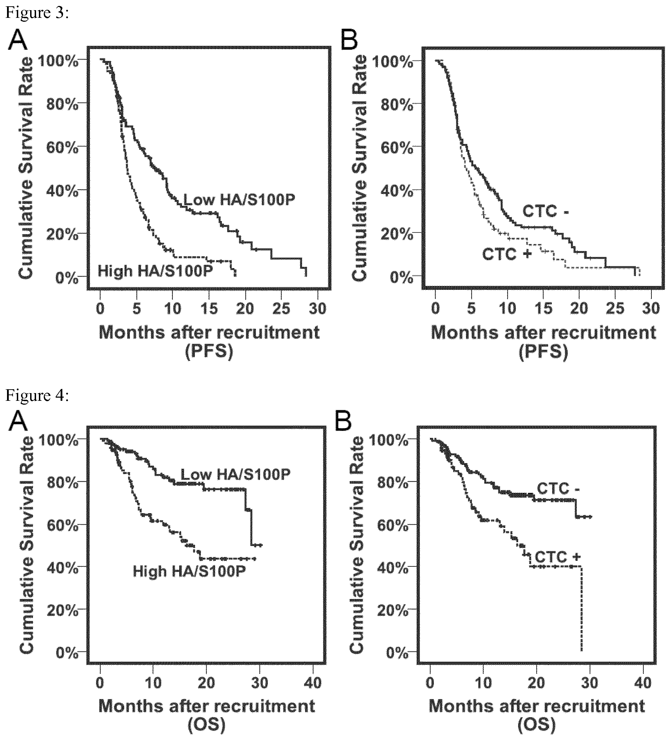

Since the plasma HA/S100P level is correlated with the CTC status which has been known as a prognostic marker of MBC, we further investigated the prognostic value of plasma HA/S100P level in MBC patients. The Progression free survival (PFS) was defined as time (in months) from recruitment to disease progression. An optimal cut off point was selected from a series of proposed plasma HA/S100P levels, at which the median PFS time of the 'positive' patients initially reaches a plateau.Using this strategy, patients presented a plasma HA level greater than 250 ng/ml and S100P level higher than 7 ng/ml were defined as 'high' HA/S100P level. As shown in Figure 3A, patients with 'low' HA/S100P level presented a more favorable outcome (median PFS time: 7,5 months, 95% CI: 5,2 - 9,8 months) than those with 'high' HA/S100P level (median PFS time: 3,7 months, 95% CI: 3,1 - 4,3 moths, log-rank test p = 2,66×10-6, Figure 3A and Table 2). In contrast, we have only observed boarder-line significant difference of PFS between CTC negative and positive patients in our study (log-rank test p = 0,082, Figure 3B and Table 2). In multivariate Cox regression model, plasma HA and S100P levels associated independently with PFS (Table 3).

**Table 2: Kaplan-Meier analysis of progression free survival with plasma HA/S100P level and CTC status.**

| Criteria | Group | N | Median PFS (95% CI) | *p* |
|---|---|---|---|---|
| HA/S100P | Low HA/S100P | 101 | 7,5 (5,2 - 9,8) | 2,66×10⁻⁶ |
| | High HA/S100P | 94 | 3,7 (3,1 - 4,3) | |
| CTC Status | CTC - | 124 | 5,6(3,8 - 7,4) | 0,082 |
| | CTC + | 71 | 4,3 (3,1 - 5,5) | |

**Table 3: Cox regression model with CTC status, plasma HA level, S100P level, and other covariates associated with progression free survival of MBC patients.**

| Prognostic factors | Univariate Cox regression | | | Multivariate Cox regression | | |
|---|---|---|---|---|---|---|
| | HR | 95% CI | *p* | HR | 95% CI | *p* |
| Metastatic sites | | | | | | |
| Lung | 1,76 | 1,28-2,43 | 5,29 × 10⁻⁴ | 1,96 | 1,40-2,76 | 9.74×10⁻⁵ |
| Liver | 1,26 | 0,92-1,72 | 0,015 | 0,99 | 0,65-1,31 | 0,648 |
| Bone | 1,07 | 0,77-1,49 | 0,702 | - | - | - |
| Skin | 1,39 | 0,92-2,10 | 0,116 | 1,46 | 0,93-2,28 | 0,099 |
| Pleura | 1,01 | 0,66-1,53 | 0,975 | - | - | - |
| Number of metastasis | 1,48 | 0,96-2,30 | 0,078 | 1,42 | 0,88-2,30 | 0,149 |
| *Multiple vs, Single* | | | | | | |
| Disease free interval | 0,93 | 0,68-1,27 | 0,633 | - | - | - |
| *> 2 years vs,* ≤ *2 years* | | | | | | |
| Age | 0,87 | 0,75-1,01 | 0,075 | 0,85 | 0,73-0,98 | 0,030 |
| *Increase by 10 years* | | | | | | |
| CTC status | 1,33 | 0,96-1,85 | 0,084 | 0,84 | 0,56-1,25 | 0,385 |
| Positive vs, Negative | | | | | | |
| **HA level** | 2,26 | 1,57-3,27 | 1,38×10⁻⁵ | 2,66 | 1,76-4,03 | 3,71×10⁻⁶ |
| ***High vs, Low*** | | | | | | |
| **S100P level** | 1,44 | 1,03-2,21 | 0,035 | 1,47 | 1,03-2,11 | 0,035 |
| ***High vs, Low*** | | | | | | |

Since HA presented a good predictive value to PFS, it might be informative to investigate its association with overall survival (OS) as well. The OS was defined as time (in months) from recruitment to death. During the follow-up period, 60 patients died of metastasis or related conditions. Kaplan-Meier analysis demonstrated that patients with higher plasma HA/S100P level had shorter OS time (median OS time: 17,5 months, 95% CI: 14,9-20,0 months), compared with those who had lower plasma HA level (median OS time: 24,6 months, 95% CI: 22,6 - 26,6 moths, log-rank test p = 7,22×10-5, Figure 4A and Table4). Meanwhile, CTC status also presented a good correlation with OS. Patients with positive CTC status had shorter OS time (median OS time: 17.0 months, 95% CI: 14,1 - 20,0months), compared with those who had negative CTC status (median OS time: 23.6 months, 95% CI: 21,7 - 25,6 moths, log-rank test p = 0,001, Figure 4B and Table 4).

**Table 4: Kaplan-Meier analysis of overall survival with plasma HA/S100P level and CTC status.**

| Criteria | Group | N | Median OS (95% CI) | *p* |
|---|---|---|---|---|
| HA/S100P | Low HA/S100P | 101 | 24,6 (22,6-26,6) | 7,22×10⁻⁵ |
| | High HA/S100P | 94 | 17,5 (14,9-20,0) | |
| CTC Status | CTC - | 124 | 23,6 (21,7-25,6) | 0,001 |
| | CTC + | 71 | 17,0 (14,1-20,0) | |

Plasma HA and S100P level was also independently associated with OS, confirmed by multivariate Cox regression model (Table 5).

**Table 5: Cox regression model with CTC status, plasma HA level, S100P level, and other covariates associated with overall survival of MBC patients.**

| Prognostic factors | Univariate Cox regression | | | Multivariate Cox regression | | |
|---|---|---|---|---|---|---|
| | HR | 95% CI | *p* | HR | 95% CI | *p* |
| Metastatic sites | | | | | | |
| **L**ung | 2,70 | 1,61-4,50 | 1,54 × 10⁻⁴ | 3,67 | 2,11-6,40 | 4.27×10⁻⁶ |
| Liver | 1,57 | 0,94-2,61 | 0,08 | 0,93 | 0,540-1,60 | 0,78 |
| Bone | 1,49 | 0,83-2,65 | 0,18 | - | - | - |
| Skin | 1,29 | 0,67-2,49 | 0,44 | - | - | - |
| Pleura | 0,96 | 0,49-1,90 | 0,92 | - | - | - |
| Number of metastasis | 3,51 | 1,27-9,73 | 0,01 | 3,31 | 1,20-9,81 | 0,03 |
| *Multiple vs. Single* | | | | | | |
| Disease free interval | 0,85 | 0,51-1,42 | 0,53 | - | - | - |
| > *2 years vs.* ≤ *2years* | | | | | | |
| Age | 0,89 | 0,70-1,13 | 0,33 | - | - | - |
| *Increase by 10 years* | | | | | | |
| CTC status | 2,35 | 1,41-3,92 | 1,02×10⁻³ | 2,15 | 1,20-3,84 | 0,01 |
| Positive vs. Negative | | | | | | |
| HA level | 4,42 | 2,00-9,72 | 2,23×10⁻⁴ | 3,78 | 1,67-8,60 | 1.51×10⁻³ |
| *High vs. Low* | | | | | | |
| S100P level | 1,99 | 1,10-3,60 | 0,02 | 1,96 | 1,05-3,64 | 0,03 |
| *High vs. Low* | | | | | | |

Thus, plasma HA/S100P level has a better predictive power to determine the progression free survival and overall survival time than the actual FDA approved CTC based test.

### Example 3: The prognostic value of the plasma HA/S100P level changes and its association to treatment outcome

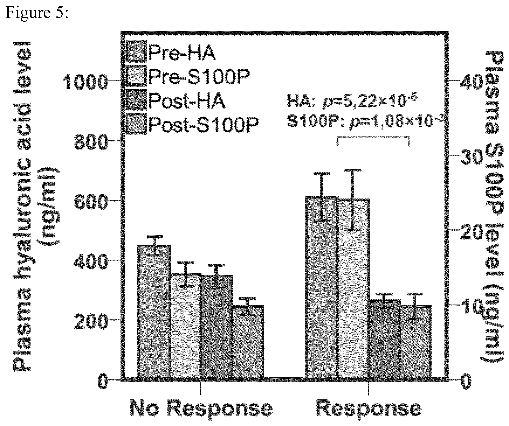

Since plasma HA/S100P level showed a correlation with progression of disease, it may have the potential to evaluate response to the anti-tumor treatment. Blood samples from 66 CTC positive MBC patients were collected before and after the first complete cycle of chemotherapy. The progress of the disease was determined by radiographic evaluation as complete remission (tumor shrink), partly remission (tumor shrink by at least 50% of baseline examination), stable disease (tumor size stable) or progressed disease (tumor size increased or metastasis at other location). Patients with progressed disease were considered 'no response' to the treatment, while the other 3 outcomes indicated that the patients 'response' to the treatment. The plasma S100P and HA level in the plasma was significantly decreased by 39% and 37% in patients who showed response to the treatment (p = 1,08×10-3 for S100P, and p = 5,22×10-5 for HA, Figure 5 and Table 6), while in the patients didn't responded the reduction rates for S100P and HA were only 19% and 17% respectively.

**Table 6: Plasma HA/S100P level in MBC patients before and after 1 complete cycle of chemotherapy.**

| Outcome | Indicator | Before treatment (median, IQR) | After treatment (median, IQR) | Difference % | *p* |
|---|---|---|---|---|---|
| No reponse | HA | 409,9 (319,3-522,2) | 313,6 (230,6-402,5) | 17% | 0,75 |
| | S100P | 11,6 (7,7-18,6) | 9,8 (4,7-12,9) | 19% | 0,01 |
| Response | HA | 438,6 (296,6-596,1) | 197,0 (162,6-307,8) | 37% | 5,22×10⁻⁵ |
| | S100P | 16,4 (10,3-43,2) | 7,9 (4,7-15,8) | 39% | 1,08×10⁻³ |

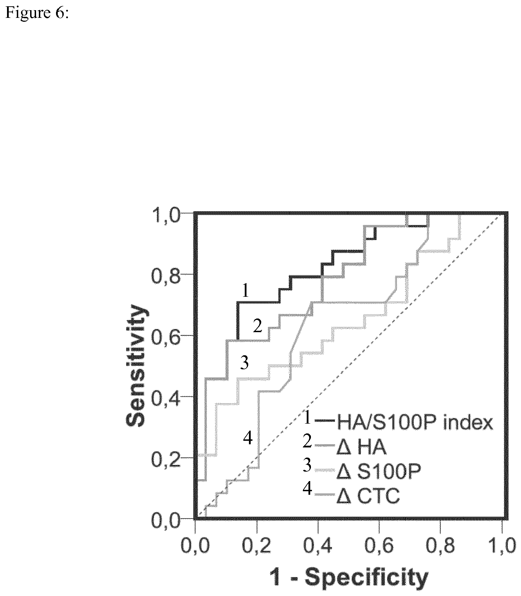

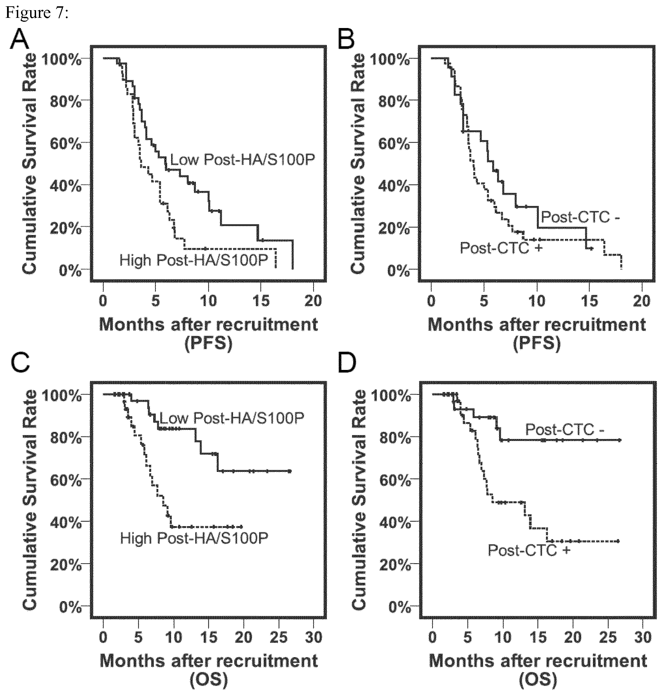

A HA/S100P index was constructed by logistic regression model and was applied for indication of radiographic response. The HA/S100P reduction index presented a larger area under the curve (AUC = 0.82, 95% CI: 0.71-0.93) compared with CTC reduction (AUC = 0.63, 95% CI: 0.48-0.78, Figure 6). Meanwhile, this HA/S100P index had a better coincidence with radiographic outcome than single markers. The prognostic value of plasma HA/S100P level after treatment was subsequently confirmed by Kaplan-Meier analysis. The previously defined cutoff point, higher than 250 ng/ml HA and 7 ng/ml S100P were used to stratify these 66 patients based on their plasma HA/S100P level after chemotherapy. Compared with those who had low HA/S100P, the patients showed high HA/S100P level even after treatment presented a shorter PFS (median PFS time: 3,6 months vs. 5,9 months, Log Rank test p=0,02, Figure 7A and Table 7).

**Table 7: Kaplan-Meier analysis of progression free survival with after treatment plasma HA/S100P level and CTC status.**

| Criteria | Group | N | Median PFS (95% CI) | *p* |
|---|---|---|---|---|
| HA/S100P | Low Post-HA/S100P | 37 | 5,9 (2,7-9,1) | 0,02 |
| | High Post-HA/S100P | 29 | 3,6 (2,4-4,8) | |
| CTC Status | Post-CTC - | 29 | 6,3 (3,4-9,2) | 0,1 |
| | Post-CTC + | 37 | 4,0 (3,4-4,6) | |

Similarly, these patients also showed significantly shorter OS (median OS time: 11,2 months vs. 20,9 months, Log Rank test p=2,92×10-3, Figure 7C and Table 8). The post-treatment CTC numbers showed a very weak association with PFS, which is not statistically significant (Log Rank test p=0,1, Figure 7B and Table 7), but it has a better prognostic value to overall survival (Log Rank test p=4,87×10-3, Figure 7D and Table 8).

**Table 8: Kaplan-Meier analysis of overall survival with after treatment plasma HA/S100P level and CTC status.**

| Criteria | Group | N | Median OS (95% CI) | *p* |
|---|---|---|---|---|
| HA/S100P | Low Post-HA/S100P | 37 | 20,9 (17,6-24,2) | 2,9×10⁻³ |
| | High Post-HA/S100P | 29 | 11,2 (8,4-14,0) | |
| CTC Status | Post-CTC | 29 | 22,2 (18,8-25,6) | 4,9×10⁻³ |
| | Post-CTC + | 37 | 13,8 (10,3-17,5) | |

In conclusion, plasma HA/S100P level is associated with the metastatic process of BC and may serve as a potential marker for the detection of metastasis. The combination of plasma HA and S100P provides a prognostic marker for the progression free survival of MBC patients, and its changes may indicate the outcome of anti-cancer treatment.

### Example 4: Prognostic Value of HA as Single Biomarker

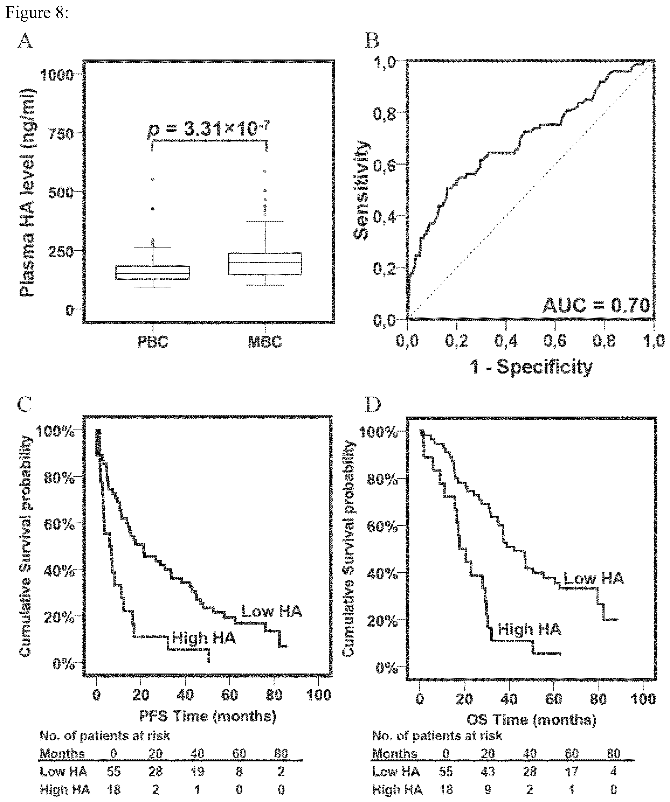

To further validate its prognostic value, the plasma HA level was measured in an independent population-based cohort consisting of 261 PBC and 73 MBC patients. In the validation cohort, higher plasma HA levels were observed in MBC patients than in PBC patients (p = 3.07×10-7, Figure 8A). Similarly to the discovery study, the plasma HA levels in the validation study showed no correlation with clinicopathological characteristics of the tumor, in PBC patients, while in MBC patients the elevated plasma HA levels were observed in patients with positive nodal status and negative estrogen receptor (ER) status. In the validation cohort, the plasma HA level also showed the discriminatory power to distinguish MBC patients from PBC patients (AUC = 0.70, 95% CI: 0.62-0.77, Figure 8B).

In the validation cohort, 73 MBC patients were followed up for possible disease progression or death after their first diagnosis, so as to record their PFS and OS. The previously defined cutoff point of 250 ng/mL was again applied to determine whether a patient had a 'high' or 'low' plasma HA level and further validate the prognostic value of plasma HA level. After a median follow-up time of 34.6 months, patients with low plasma HA levels experienced a significantly longer PFS and OS than those with high plasma HA levels (median PFS time: 21.1 vs. 5.8 months, log-rank test p = 3.66×10-4; median OS time: 42.2 vs. 17.6 months, log-rank test p = 1.43×10-4, Figure 8C, 8D).

## Claims

1. Method for the diagnosis, prognosis, stratification and/or monitoring of a therapy, of breast cancer in a patient, comprising the steps of
a. Providing a blood sample of said patient,
b. Determining the level of hyaluronic acid (HA) and S100P in said blood sample, and
c. Comparing the level of HA and S100P as determined in b. with a control,
wherein an increase in the level of HA and S100P in said blood sample compared to said control is indicative for the presence of breast cancer in said patient.

2. The method according to claim 1, wherein the breast cancer is metastatic breast cancer or recurrent breast cancer, or circulating tumor cell (CTC) positive breast cancer.

3. The method according to any one of the preceding claims, wherein said control corresponds to the level of HA and S100P in a control sample from a healthy individual, or a control sample from non-metastatic breast cancer patient, or a control sample from a CTC negative breast cancer patient.

4. A method for evaluating the treatment success of a breast cancer patient who received a breast cancer treatment, comprising
a. Providing a blood sample of said breast cancer patient who received a breast cancer treatment,
b. Determining the level of HA and S100P in said blood sample, and
c. Comparing the level of HA and S100P as determined in b. with a reference,
wherein a decrease of at least 20%, preferably 25%, more preferably 30%, most preferably 35% to 45% of the level of HA and S100P in said blood sample compared to said reference is an indication for the patient's response to said treatment.

5. The method according to claim 4, wherein said reference corresponds to the level of HA and S100P in a provided blood sample obtained from said patient before receiving said treatment.

6. A method for the prognosis of a breast cancer patient, comprising
a. Providing a blood sample of said breast cancer patient,
b. Determining the level of HA and S100P in said blood sample,
wherein an elevated level of HA and S100P in said blood sample is an indication for a reduced time of progression free survival (PFS) and/or overall survival (OS).

7. The method according to any one of the preceding claims, wherein said blood sample is a plasma or serum sample.

8. The method according to any one of the preceding claims, wherein said patient is a mammal, preferably a human, more preferably a human patient diagnosed with breast cancer, or a human patient diagnosed with metastatic breast cancer.

9. The method according to any one of the preceding claims, wherein the level of HA and S100P in said sample is determined by means of a detection method selected from the group consisting of mass spectrometry, mass spectrometry immunoassay (MSIA), antibody-based protein chips, 2-dimensional gel electrophoresis, stable isotope standard capture with anti-peptide antibodies (SISCAPA), high-perfomrance liquid chromatography (HPLC), western blot, cytometry bead array (CBA), protein immuno-precipitation, radio immunoassay, ligand binding assay, and enzyme-linked immunosorbent assay (ELISA), preferably wherein said protein detection method is ELISA.

10. A diagnostic kit for performing a method according to any one of the preceding claims, the kit comprising means for quantifying the level of HA and S100P.

11. Use of HA and S100P as biomarker in a screening method for breast cancer therapeutics.

## Patentansprüche

1. Verfahren zur Diagnose, Prognose, Stratifizierung und/oder Überwachung einer Therapie für Brustkrebs bei einem Patienten, umfassend die Schritte
a. Bereitstellen einer Blutprobe des Patienten,
b. Bestimmen des Spiegels von Hyaluronsäure (HA) und S100P in der Blutprobe, und
c. Vergleichen des Spiegels von HA und S100P wie in b. bestimmt mit einer Kontrolle,
wobei eine Erhöhung des Spiegels von HA und S100P in der Blutprobe verglichen mit der Kontrolle das Vorhandensein von Brustkrebs in dem Patienten anzeigt.

2. Das Verfahren nach Anspruch 1, wobei der Brustkrebs metastasierender Brustkrebs oder rezidivierender Brustkrebs oder zirkulierender Tumorzellen (CTC)-positiver Brustkrebs ist.

3. Das Verfahren nach einem der vorhergehenden Ansprüche, wobei die Kontrolle dem Spiegel von HA und S100P in einer Kontrollprobe von einem gesunden Individuum oder einer Kontrollprobe von einem nicht-metastasierendem Brustkrebs-Patienten oder einer Kontrollprobe von einem CTC-negativen Brustkrebspatienten entspricht.

4. Verfahren zur Bewertung des Behandlungserfolges eines Brustkrebspatienten, der eine Brustkrebsbehandlung erhalten hat, umfassend
a. Bereitstellen einer Blutprobe des Brustkrebspatienten, der eine Brustkrebsbehandlung erhalten hat,
b. Bestimmen des Spiegels von HA und S100P in der Blutprobe, und
c. Vergleichen des Spiegels von HA und S100P, wie in b. bestimmt mit einer Referenz,
wobei eine Abnahme von mindestens 20%, vorzugsweise 25%, mehr bevorzugt 30%, besonders bevorzugt 35% bis 45% des Spiegels von HA und S100P in der Blutprobe im Vergleich zur Referenz eine Indikation für die Reaktion des Patienten auf diese Behandlung ist.

5. Das Verfahren nach Anspruch 4, wobei die Referenz dem Spiegel von HA und S100P in einer bereitgestellten Blutprobe entspricht, die von dem Patienten vor dem Erhalten der Behandlung erhalten wurde.

6. Verfahren zur Prognose eines Brustkrebspatienten, umfassend
a. Bereitstellen einer Blutprobe des Brustkrebspatienten,
b. Bestimmen des Spiegels von HA und S100P in der Blutprobe,
wobei ein erhöhter Spiegel von HA und S100P in der Blutprobe ein Hinweis auf eine verringerte Zeit des progressionsfreien Überlebens (PFS) und/oder des Gesamtüberlebens (OS) ist.

7. Das Verfahren nach einem der vorhergehenden Ansprüche, wobei die Blutprobe eine Plasma- oder Serumprobe ist.

8. Das Verfahren nach einem der vorhergehenden Ansprüche, wobei der Patient ein Säuger, vorzugsweise ein Mensch, besonders bevorzugt ein mit Brustkrebs diagnostizierter menschlicher Patient oder ein mit metastasierendem Brustkrebs diagnostizierter menschlicher Patient ist.

9. Das Verfahren nach einem der vorhergehenden Ansprüche, wobei der Spiegel von HA und S100P in der Probe mittels eines Nachweisverfahrens bestimmt wird, welches ausgewählt wird aus der Gruppe bestehend aus Massenspektrometrie, Massenspektrometrie-Immunoassay (MSIA), Antikörper-basierte Protein-Chips, 2-dimensionale Gelelektrophorese, Fest-Isotopen-Standard-Capture mit Anti-Peptid-Antikörpern (SISCAPA), Hochleistungs-Flüssigkeitschromatographie (HPLC), Western Blot, Zytometrie-Bead-Array (CBA), Proteinimmunpräzipitation, Radioimmunoassay, Ligandbindungstest und enzymgekoppeltem Immunadsorptionstest (ELISA), wobei das Proteinnachweisverfahren vorzugsweise ELISA ist.

10. Ein diagnostisches Kit zur Durchführung eines Verfahrens nach einem der vorhergehenden Ansprüche, wobei das Kit Mittel zur Quantifizierung des Gehalts von HA und S100P umfasst.

11. Verwendung von HA und S100P als Biomarker in einem Screening-Verfahren nach Brustkrebs-Therapeutika.

## Revendications

1. Procédé pour le diagnostic, le pronostic, la stratification et/ou la surveillance d'un traitement, d'un cancer du sein chez un patient, comprenant les étapes suivantes :
a. obtention d'un échantillon de sang dudit patient,
b. détermination des niveaux d'acide hyaluronique (HA) et de S100P dans ledit échantillon de sang, et
c. comparaison des niveaux de HA et de S100P tels que déterminé en b. avec un témoin,
dans lequel une augmentation des niveaux de HA et de S100P dans ledit échantillon de sang, par rapport audit témoin, est indicative de la présence d'un cancer du sein chez ledit patient.

2. Procédé selon la revendication 1, dans lequel le cancer du sein est un cancer du sein métastasique ou un cancer du sein récurrent, ou un cancer du sein positif à des cellules tumorales en circulation (CTC).

3. Procédé selon l'une quelconque des revendications précédentes, dans lequel ledit témoin correspond aux niveaux de HA et de S100P dans un échantillon témoin provenant d'un individu en bonne santé, ou d'un échantillon témoin provenant d'un patient ayant un cancer du sein non métastasique, ou d'un échantillon témoin provenant d'un patient ayant un cancer du sein négatif aux CTC.

4. Procédé pour évaluer le succès du traitement d'un patient ayant un cancer du sein qui a reçu un traitement contre un cancer du sein, comprenant :
a. l'obtention d'un échantillon de sang dudit patient ayant un cancer du sein qui a reçu un traitement contre un cancer du sein,
b. la détermination des niveaux de HA et de S100P dans ledit échantillon de sang, et
c. la comparaison des niveaux de HA et de S100P tels que déterminé en b. avec une référence,
dans lequel une diminution d'au moins 20 %, de préférence 25 %, mieux encore 30 %, tout spécialement 35 % à 45 % des niveaux de HA et de S100P dans ledit échantillon de sang par rapport à ladite référence est une indication de la réponse du patient audit traitement.

5. Procédé selon la revendication 4, dans lequel ladite référence correspond aux niveaux de HA et de S100P dans un échantillon de sang fourni obtenu auprès dudit patient avant réception dudit traitement.

6. Procédé pour le pronostic d'un patient ayant un cancer du sein, comprenant :
a. l'obtention d'un échantillon de sang dudit patient ayant un cancer du sein,
b. la détermination des niveaux de HA et de S100P dans ledit échantillon de sang,
dans lequel des niveaux élevés de HA et de S100P dans ledit échantillon de sang constituent une indication d'un temps réduit de la survie sans progression (FPS) et/ou de la survie globale (OS).

7. Procédé selon l'une quelconque des revendications précédentes, dans lequel ledit échantillon de sang est un échantillon de plasma ou de sérum.

8. Procédé selon l'une quelconque des revendications précédentes, dans lequel ledit patient est un mammifère, de préférence un humain, mieux encore un patient humain auquel a été diagnostiqué un cancer du sein, ou un patient humain auquel a été diagnostiqué un cancer du sein métastasique.

9. Procédé selon l'une quelconque des revendications précédentes, dans lequel les niveaux de HA et de S100P dans ledit échantillon sont déterminés au moyen d'un procédé de détection choisi dans le groupe constitué par une spectrométrie de masse, un immunodosage avec spectrométrie de masse (MSIA), des puces à protéine à base d'anticorps, une électrophorèse sur gel à deux dimensions, une capture standard d'isotopes stables avec des anticorps anti-peptides (SISCAPA), une chromatographie liquide haute performance (HPLC), un transfert Western, un réseau de perles de cytométrie (CBA), une immunoprécipitation de protéines, un radioimmunodosage, un dosage de liaison de ligands, et un immunodosage enzymatique (ELISA), de préférence dans lequel ledit procédé de détection de protéine est un ELISA.

10. Trousse de diagnostic pour la mise en oeuvre d'un procédé selon l'une quelconque des revendications précédentes, la trousse comprenant des moyens pour quantifier les niveaux de HA et de S100P.

11. Utilisation de HA et de S100P en tant que marqueurs biologiques dans un procédé de criblage de substances thérapeutiques contre un cancer du sein.
